# EUROPEAN PATENT APPLICATION

(11) **EP 4 269 519 A1**
(43) Date of publication of application: **01.11.2023**
(21) Application number: 21911067.3
(22) Date of filing: 24.12.2021
(51) Int. Cl.: C09J 5/00, G01N 37/00, C09J 201/00, G01N 35/08

(54) **METHOD FOR MANUFACTURING MICRO CHIP FOR LIQUID SAMPLE ANALYSIS**

(30) Priority: 24.12.2020 JP 2020215587
(71) Applicant: Fujimori Kogyo Co., Ltd., Tokyo 112-0002 (JP)
(72) Inventor: ABE, Masato, Tokyo 112-0002 (JP); WADA, Tomoko, Tokyo 112-0002 (JP); SATO, Takao, Tokyo 112-0002 (JP); HOSOKAWA, Kazuya, Tokyo 112-0002 (JP)
(74) Representative: Jones, Nicholas Andrew
(86) International application number: PCT/JP2021/048238
(87) International publication number: WO 2022/138930

(57) **Abstract**

Provided is a method for manufacturing a micro chip which is for liquid sample analysis and has a flow path therein, the method being characterized by comprising: a step for preparing a base material (1) having, in a surface thereof, a groove serving as a flow path; a step for preparing a film (2) which covers the surface of the base material (1) to seal the groove and can thus form a flow path; a step for applying, on the base material (1), an adhesive (3) and/or a sticking agent (3); and a step for attaching the film (2) onto the base material (1) so that a surface of the base material (1), to which the adhesive (3) and/or the sticking agent (3) is applied, and the film (2) overlap each other, wherein the attaching step includes a step for pressure-bonding the film (2) and the base material (1) under 32-643 kPa, and a step for curing the adhesive (3) and/or the sticking agent (3).

## Description

### TECHNICAL FIELD

The present invention relates to a method of producing a microchip for liquid sample analysis.

### BACKGROUND ART

It is known that a component in a liquid sample such as blood can be analyzed by introducing the liquid sample into a flow path in a microchip, and reacting the sample with an antibody or the like in a reaction section provided in the middle of the flow path.

In a known method for the preparation of such a microchip, a film is attached using an adhesive agent to a substrate having a surface on which a groove corresponding to the flow path is formed (Patent Document 1 or 2).

However, the method has problems in, for example, that insufficient adhesion between the substrate and the film may occur due to formation of a projection (protrusion) on the surface of the microchip during molding of the microchip, which insufficient adhesion leads to leakage of a liquid during the use.

### PRIOR ART DOCUMENTS

### [Patent Documents]

Patent Document 1: JP 2008-232939 A
Patent Document 2: JP 2008-175795 A

### SUMMARY OF THE INVENTION

### PROBLEMS TO BE SOLVED BY THE INVENTION

An object of the present invention is to provide a method of manufacturing a microchip having a flow path therein by attaching a film to a substrate, wherein adhesion between the film and the substrate can be achieved even when there is a projection on the surface of the substrate, which microchip hence does not suffer from liquid leakage.

### MEANS FOR SOLVING THE PROBLEMS

The present inventors intensively studied in order to solve the above problem. As a result, the present inventors discovered that, in a method of manufacturing a microchip for liquid sample analysis comprising a flow path therein, wherein a substrate having a surface on which a groove corresponding to a flow path is formed is attached using an adhesive agent and/or a gluing agent to a film capable of forming the flow path by covering the substrate surface to seal the groove, a microchip in which the film is adhering without forming a gap and which hence does not suffer from liquid leakage even when there is a projection on the substrate surface can be simply produced by carrying out the steps of pressing the film onto the substrate and curing the adhesive agent and/or a gluing agent. The present inventors also discovered that highly accurate analysis of a liquid sample can be carried out using the obtained microchip. Further, the present inventors discovered a member, a pressure required for the pressing, and a length of time for which the pressure is to be maintained, for achieving efficient attachment between the substrate and the film, thereby completing the present invention.

More specifically, the present invention provides a method for manufacturing a microchip for liquid sample analysis, the microchip comprising a flow path therein, the method comprising:
a step of providing a substrate comprising on a surface thereof a groove serving as the flow path;
a step of providing a film capable of forming the flow path by covering the surface of the substrate to seal the groove;
a step of applying an adhesive agent and/or a gluing agent to the surface of the substrate on which the groove is formed; and
a step of attaching the film on the substrate in such a manner that the film and the surface of the substrate on which the adhesive agent and/or the gluing agent is/are applied overlap each other; and
wherein the step of attaching comprises
   a step of pressing the film onto the substrate at 32 to 643 kPa, and
   a step of curing the adhesive agent and/or the gluing agent.

In one embodiment of the present invention, the step of pressing the film onto the substrate may be carried out by use of a member in which a cavity is formed, the member having a film-contacting surface comprising an elastic material, which film-contacting surface inflates when a gas is injected from one end of the cavity, wherein the film-contacting surface of the member is brought into contact with the film surface of the microchip, and a gas is injected into the member to apply pressure onto the film surface of the microchip. The member in which a cavity is formed may be a member prepared by attaching a plate-like member to a sheet comprising an elastic material for covering the film surface, such that a gas can be injected into the member.

In another embodiment of the present invention, the step of pressing the film onto the substrate may be carried out by applying pressure to an auxiliary sheet comprising an elastic material for covering the film surface, the auxiliary sheet being placed in the film side.

The elastic material is preferably rubber or silicone.

The adhesive agent and/or the gluing agent may be a photocurable adhesive agent and/or a photocurable gluing agent, such as a UV-curable adhesive agent and/or a UV-curable gluing agent, and the member in which a cavity is formed or the auxiliary sheet used in the attachment step may be constituted by an optically transparent material such as a UV-transparent material.

### EFFECT OF THE INVENTION

According to the present invention, a microchip for liquid sample analysis can be simply and inexpensively produced. By carrying out the steps of pressing the film onto the substrate at a certain pressure, and curing the adhesive agent and/or the gluing agent, a microchip in which the film is adhering without forming a gap and which hence does not suffer from liquid leakage even when there is a projection on the substrate surface can be simply produced. Highly accurate analysis of a liquid sample can be carried out using the obtained microchip. Further, uniform pressing of the substrate onto the film can be carried out using, as a member for pressing the substrate onto the film, for example, a member in which a cavity is formed, the member having a film-contacting surface comprising an elastic material, which film-contacting surface inflates when a gas is injected from one end of the cavity; or an auxiliary sheet comprising an elastic material for covering the film.

### BRIEF DESCRIPTION OF THE DRAWINGS

Fig. 1A is a diagram illustrating one embodiment of a substrate of a microchip (before application of an adhesive agent).
Fig. 1B is a diagram illustrating one embodiment of a substrate of a microchip (after application of an adhesive agent).
Fig. 1C is a diagram illustrating one embodiment of a film of a microchip.
Fig. 1D is a diagram illustrating one embodiment of a microchip.
Fig. 1E is a diagram illustrating a substrate of the microchip of Fig. 1A prepared using a mold insert. The dotted line indicates a step formed by the insert.
Fig. 2 is a schematic diagram illustrating a case where a film is attached to a substrate having a flat surface, a case where a film is attached to a substrate having a projection on the surface, and a case where a film is attached to a substrate having a projection on the surface while pressing the film onto the substrate.
Fig. 3 is a schematic diagram illustrating one example of an embodiment of bonding a film to a substrate, wherein a film-contacting surface of a member in which a cavity is formed is pressed against the film, and air is injected into the member to press the film onto the substrate, followed, in this state, by curing an adhesive agent.
Fig. 4 is a schematic diagram illustrating one example of an embodiment of bonding a film to a substrate, wherein pressure is applied from both sides of the microchip by sandwiching the microchip between plate-like members, to thereby pressing the film onto the substrate, followed, in this state, by curing an adhesive agent.
Fig. 5 is a schematic diagram of a bonding jig to be used for attaching the film to the substrate

### DESCRIPTION OF EMBODIMENTS

The method for manufacturing a microchip for liquid sample analysis of the present invention comprises:
a step of providing a substrate comprising on a surface thereof a groove serving as the flow path;
a step of providing a film capable of forming the flow path by covering the surface of the substrate to seal the groove;
a step of applying an adhesive agent and/or a gluing agent to the surface of the substrate on which the groove is formed; and
a step of attaching the film on the substrate in such a manner that the film and the surface of the substrate on which the adhesive agent and/or the gluing agent is/are applied overlap each other; and
wherein the step of attaching comprises
   a step of pressing the film onto the substrate at 32 to 643 kPa, and
   a step of curing the adhesive agent and/or the gluing agent.

The liquid sample is not limited as long as the sample can be passed through the microchip, and examples of the liquid sample include: liquid samples obtained from living bodies, such as blood and urine, and dilutions thereof; extracts from living bodies such as plants and animals; naturally occurring waters such as those in rivers, seas, and rains; washing liquids; and waste liquids. The component in the samples is also not limited, and examples of the component include proteins, nucleic acids, low-molecular-weight compounds, sugars, and cells.

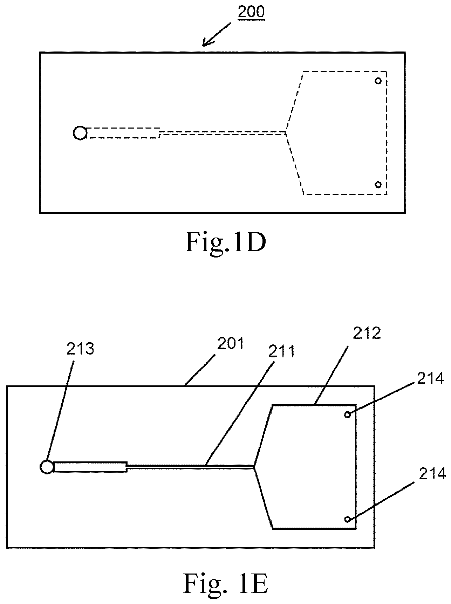

The method of producing a microchip for liquid sample analysis of the present invention is described below with reference to drawings. However, the following are merely examples, and the production method of the present invention and the microchip obtained by the method are not limited to the following modes. The microchip is not limited as long as a liquid sample can be injected into the microchip to allow the sample to flow through a flow path therein. For example, the discharge port and the reaction section in Fig. 1D are not indispensable.

Fig. 1D is a schematic diagram illustrating an example of the form of a microchip 200.

### <Substrate>

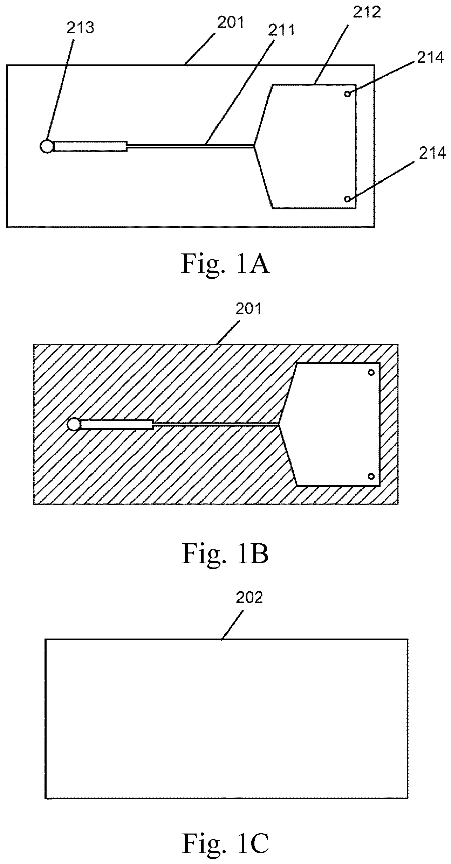

Fig. 1A is a plan view of a substrate 201 having a surface on which a groove corresponding to a flow path 211 of the microchip 200 is formed. In a first-end side of the groove, a penetrating hole corresponding to an inlet 213 for the liquid sample is provided. On the other-end side, a pit corresponding to a waste liquid storage section 212 is provided, and penetrating holes corresponding to air holes 214 are provided at two sites in the pit. The inlet and the air holes may be provided on the film side. For example, a groove corresponding to the flow path, and a pit corresponding to the waste liquid storage section may be provided on the substrate, and a film in which holes are formed at positions corresponding to both end sides of the groove may be provided and attached to the substrate. In the middle of the flwo path, in other words, on one part between the penetrating hole corresponding to the inlet and the penetrating holes corresponding to the air holes, a pit corresponding to a reaction section may be provided. Instead of the waste liquid storage section, an outlet may be provided.

The shape of the flow path is not limited, and may be either a linear shape or curved shape. The flow path may be branched. In this case, two or more each of inlets, reaction sections, and/or outlets or waste liquid storage sections may be present. For example, two inlets may be provided, and a liquid sample may be allowed to flow from a first inlet into a first flow path, and a reaction substrate liquid may be allowed to flow from a second inlet into a second flow path. A reaction section may be provided at the junction of the first flow path and second flow path, and a confluent flow path and an outlet (discharge port) may be provided downstream thereof.

The cross-sectional shape of the groove corresponding to the flow path is not limited, and may be, for example, a recessed shape, U-shape, or V-shape. The depth of the groove corresponding to the flow path is preferably 10 to 500 µm, and the width of the groove is preferably 10 µm to 3 mm. The length of the portion corresponding to the flow path is, for example, 3 mm to 5 cm.

The width of the groove may or may not be constant. The depth of the groove may or may not be constant.

The size of the pit corresponding to the waste liquid storage section is not limited as long as the liquid sample that has been introduced from the inlet and has passed through the flow path can be stored therein. The shape of the pit is also not limited, and may be, for example, a cylindrical shape or prismatic shape. By increasing the area and depth, a larger amount of liquid sample can be stored. The area of the pit is, for example, 0.1 to 50 mm². In cases of a circular reaction section, its diameter is, for example, 0.2 to 6 mm. However, the area may vary depending on the depth of the groove. For example, the pit may have a conical shape. The pit is preferably deeper than the groove corresponding to the flow path, and has a depth of, for example, 20 µm to 3 mm.

When a pit corresponding to the reaction section is provided, its area and depth may also be as described above.

The size of the penetrating hole corresponding to the inlet 213 is not limited as long as a liquid sample such as blood can be injected therethrough using a microsyringe or the like. Its diameter is, for example, 0.2 to 3 mm.

The size of each penetrating hole corresponding to an air hole 214 is not limited as long as the hole can function as an air hole. Its diameter is, for example, 0.2 to 2 mm.

In cases where a pit corresponding to the reaction section is provided in the middle of the flow path, a reagent and/or the like may be placed in the reaction section.

The size of the microchip is not limited as long as the groove corresponding to the flow path, and the pit(s) corresponding to the reaction section and/or waste liquid storage section can be formed on the surface. The thickness of the microchip is also not limited, and may be, for example, 0.5 to 5 mm.

As a material of the substrate of the microchip, a metal, glass, plastic, silicone, or the like may be used. From the viewpoint of detecting the reaction by luminescence, coloring, or visual observation, and from the viewpoint of curing the adhesive agent and/or a gluing agent using a light such as ultraviolet, the material is preferably a transparent material through which these lights and the like can pass. A transparent plastic is more preferred. Examples of the material include polyethylene, polypropylene, polystyrene, polymethyl methacrylate, cycloolefin polymers, cycloolefin copolymers, polyphenylene oxide, polyethylene terephthalate, polyethylene naphthalate, polycarbonate, polyamide, polyimide, phenolic resins, epoxy resins, polyvinylidene chloride, polyvinyl chloride, acrylonitrile-butadiene-styrene (ABS) resins, and poly 2-methoxyethyl acrylate (PMEA) resins.

The groove and the holes to be provided on the substrate of the microchip may be formed using a cutter or laser beam, or may be formed by injection molding when the material of the microchip is a plastic. In cases of the formation by injection molding, microchips having uniform quality can be efficiently prepared, which is preferred. As shown in Fig. 1E, regarding the mold to be used for the injection molding, a mold insert to be inserted as a separate part into a mold body called mother mold is preferably prepared in a trial stage for studying various flow path shapes, from the viewpoint of reducing the production cost for part replacement.

### <Film>

Fig. 1C is a plan view of a film 202. The material of the film is preferably a transparent plastic, and examples of the material include those described above. More preferred examples of the material include polyethylene terephthalate (PET) resins, cyclic olefin polymer (COP) resins, cyclic olefin copolymer (COC) resins, polystyrene (PS) resins, polycarbonate (PC) resins, and polymethyl(meth)acrylate (PMMA) resins.

The size of the film may be the same as the size of the substrate, and is not limited as long as the film can cover the flow path.

The thickness of the film is, for example, preferably 50 to 200 µm, more preferably 100 to 200 µm.

An area on the film that overlaps with part of the flow path 211 when the film is layered on the substrate 201 may be coated with a reactive substance. In cases where a pit corresponding to the reaction section is provided on the substrate surface, a position on the film that overlaps with the reaction section is preferably coated with a reactive substance.

The reactive substance is not limited as long as the substance reacts with a target component (detection target) in the liquid sample, and may be appropriately selected depending on the type of the target substance. Examples of the reactivity of the reactive substance include biological reactions and chemical reactions, and the biological reactions include binding reactions. Examples of the reactive substance include proteins (including peptides), sugars, nucleic acids, and low-molecular-weight compounds. Specific examples of the reactive substance include: substances that specifically bind to the target substance, such as antibodies; enzyme proteins using the target substance as a substrate; and blood coagulation factors such as tissue thromboplastin reagents. In cases where the target substance is a nucleic acid, the reactive substance may be a nucleic acid probe, a polymerase that amplifies the nucleic acid (nucleic acid amplification enzyme), or the like.

Fig. 1D is a plan view of a microchip 200 obtained by attaching a substrate 201 to a film 202 such that the surface of the substrate 201 on which the groove is formed is attached the film 202. The dotted line indicates that a flow path 211, a waste liquid storage section 212, and the like are present in the microchip 200.

By layering and attaching the film 202 onto the substrate 201, the top portion of the groove and the pit corresponding to the flow path and the waste liquid storage section is covered with the film, to form the flow path through which the liquid sample passes and the waste liquid storage section.

By the layering of the film, one side of each penetrating hole is sealed, and an opening remains only on the side where the film is not layered on the substrate. By this, each penetrating hole functions as an inlet or an air hole.

Thus, the liquid sample introduced from the inlet passes through the flow path, and is then stored in the waste liquid storage section. The target substance in the sample can be measured by measuring the passing speed in the flow path, or by observing or detecting a reaction in the reaction section in cases where the reaction section is provided in the middle of the flow path. Examples of the reaction herein include, but are not limited to, coloring reaction, luminous reaction, amplification reaction, and agglutination reaction.

### <Adhesive agent Application Step>

An adhesive agent and/or a gluing agent is/are used for the attachment of the film 202 to the substrate 201. Examples of the adhesive agent and/or the gluing agent include a photocurable adhesive agent and/or a photocurable gluing agent; and a thermosetting adhesive agent and/or a thermosetting gluing agent.

Examples of the adhesive agent include (meth)acrylate resin adhesives, natural rubber adhesives, urethane resin adhesives, ethylene-vinyl acetate resin emulsion adhesives, ethylene-vinyl acetate resin adhesives, epoxy resin adhesives, vinyl chloride resin solvent adhesives, chloroprene rubber adhesives, cyanoacrylate adhesives, silicone adhesives, styrene-butadiene rubber solvent adhesives, nitrile rubber adhesives, nitrocellulose adhesives, phenol resin adhesives, modified silicone adhesives, polyester adhesives, polyamide adhesives, polyimide adhesives, olefin resin adhesives, vinyl acetate resin emulsion adhesives, polystyrene resin solvent adhesives, polyvinyl alcohol adhesives, polyvinylpyrrolidone resin adhesives, polyvinyl butyral adhesives, polybenzimidazole adhesives, poly(meth)acrylate resin solvent adhesives, melamine resin adhesives, urea resin adhesives, and resorcinol adhesives. The adhesives may be used individually or as a mixture of two or more thereof.

Examples of the gluing agent include rubber glues, (meth)acrylate glues, silicone glues, urethane glues, vinyl alkyl ether glues, polyvinyl alcohol glues, polyvinyl pyrrolidone glues, polyacrylamide glues, and cellulose glues. Such gluing agents may be used individually or as a mixture of two or more thereof.

The adhesive agent or gluing agent is preferably photocurable (either radical-reactive or cationic-polymerizable), more preferably UV-curable. In cases where a UV-curable adhesive agent or UV-curable gluing agent is used, curing reaction can be quickly initiated by UV irradiation after the application step, to enable the bonding. The UV-curable adhesive agent is more preferably an acrylic UV-curable adhesive agent, such as UVX-8204 (Denka Company Limited), UVX-8400 (Denka Company Limited), SX-UV100A (Cemedine Co., Ltd.), SX-UV200 (Cemedine Co., Ltd.), BBX-UV300 (Cemedine Co., Ltd.), U-1340 (Chemitech Inc.), U-1455B (Chemitech Inc.), U-1558B (Chemitech Inc.), Aronix UV-3000 (Toagosei Co., Ltd.), TB3094 (ThreeBond Co., Ltd.), or Hitaloid 7975D (Hitachi Chemical Company, Ltd.). The UV-curable gluing agent is more preferably an acrylic UV-curable gluing agent, such as UV-3630ID80 (Mitsubishi Chemical Corporation), UX-3204 (Nippon Kayaku Co., Ltd.), or Finetack RX-104 (DIC Corporation). An acrylic UV-curable adhesive agent or acrylic UV-curable gluing agent shows favorable adhesion to a wide range of plastic materials, and is capable of quick production of strength after UV irradiation. The viscosity of the adhesive agent or gluing agent used for the attachment of the film 2 to the substrate 1 is preferably, for example, 2000 to 31,000 mPa·s.

Examples of the thermosetting adhesive agent include KE-1820 (Shin-Etsu Chemical Co., Ltd.), SA2410HB (Dexerials Corporation), AS-300213 (Asec Co., Ltd.), and EP160 (Cemedine Co., Ltd.).

For areas that do not affect the shapes of the groove and the pit corresponding to the flow path and the reaction section, the attachment strength can be increased without affecting the shapes of the groove and the pit corresponding to the flow path and the reaction section, by, for example, welding the film 202 to the substrate 201 by laser light irradiation

A description is given below for a case using an adhesive agent. However, the following also applies to a case where a gluing agent is used instead of the adhesive agent. Thus, the term "adhesive agent" may be replaced by the term "adhesive agent and/or gluing agent".

The adhesive agent is applied at a position other than the groove on the substrate surface. For example, as illustrated in Fig. 1B, the adhesive agent is preferably applied to an area other than the flow path and the reaction section on the substrate surface. For more accurate application to an area other than the groove, the adhesive agent is preferably applied by a printing technique, especially preferably by screen printing. In cases where screen printing is used, even when the adhesive agent is filled into a plate for the entire surface area of the substrate, transfer of the adhesive agent does not occur to the groove, which is not in contact with the screen printing plate, while the adhesive agent is transferred to the area other than the groove, which area is in contact with the screen printing plate. More preferably, the adhesive agent is applied such that a margin of not less than about 200 µm, preferably 200 µm to 400 µm, is secured around the groove so as to avoid entrance of the adhesive agent into the flow path during the attachment. By this, favorable application of the adhesive agent to the area other than the groove can be achieved.

The film thickness of the applied adhesive agent is preferably 5 to 15 µm. For the control of the film thickness of the adhesive agent, the number of meshes per inch in the screen is, for example, preferably 400 to 730. The mesh opening ratio is, for example, preferably 39 to 49%.

Other examples of the method of application of the adhesive agent to the substrate, which method enables accurate application of the adhesive agent to the outside of the flow path, include inkjet printing and gravure printing, and use of a dispenser.

The application of the adhesive agent may be carried out after hydrophilization treatment of the surface of the substrate. The hydrophilization treatment is preferably application of a hydrophilizing reagent, plasma treatment, corona treatment, or excimer treatment. Examples of the hydrophilizing reagent include: nonionic surfactants such as S-1570 (sucrose fatty acid ester: Mitsubishi-Chemical Foods Corporation), LWA-1570 (sucrose lauric acid ester: Mitsubishi-Chemical Foods Corporation), POEM DL-100 (diglycerin monolaurate: Riken Vitamin Co., Ltd.), and RIKEMAL A (sucrose fatty acid ester: Riken Vitamin Co., Ltd.); CeraAqua NS235-N1 (Shima Trading Co., Ltd.); AMINOION (Nippon Nyukazai Co., Ltd.); LAMBIC-771W (Osaka Organic Chemical Industry Ltd.); LAMBIC-1000W (Osaka Organic Chemical Industry Ltd.); SPRA-101 (Tokyo Ohka Kogyo Co., Ltd.); and SPRA-202 (Tokyo Ohka Kogyo Co., Ltd.). Specific examples of the treatment conditions include those in which the contact angle of water on the substrate surface becomes, for example, 40 to 55°.

The hydrophilization treatment prevents the substrate from repelling the adhesive agent, and allows wet spreading of the adhesive agent on the substrate, so that favorable attachment is possible.

### <Attachment Step>

The step of attaching the substrate to the film includes a step of pressing the substrate onto the film, and a step of curing the adhesive agent.

### <Pressing Step>

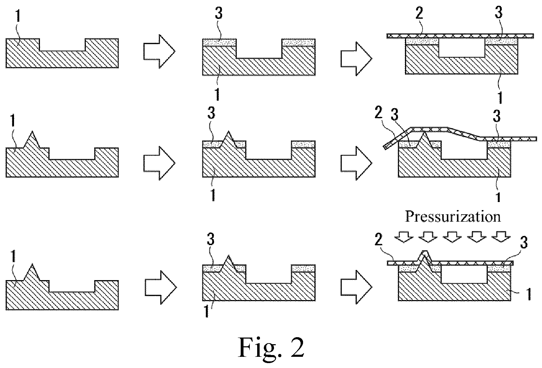

As illustrated in Fig. 2, when a microchip is produced by attaching a substrate to a film, no problem occurs in cases where the substrate is flat as in Fig. 2 (top row). However, in cases where the suubstrate surface has a projection (protruding portion) as in Fig. 2 (middle row), a loose part is formed during the attachment, and this causes liquid leakage during the use, so that an accurate test cannot be carried out. Further, in cases where the substrate is formed by injection molding utilizing the insert method, a projection or step is formed in the border between the mother mold of the substrate and the mold insert, which may lead to formation of a loose part during the attachment. As long as the mother mold and the insert are designed such that their border is distant from the flow path, the loose part formed by the mold insert may not deteriorate accuracy of the test in the flow path, but there is still a concern that a decrease in the adhering area between the substrate and the film may lead to a decreased peel strength. Thus, in order to solve these problems, in the method of the present invention, the film is pressed onto the substrate as illustrated in Fig. 2 (bottom row), and the adhesive agent is cured in this state. By this, adhesion between the film and the substrate can be achieved, and liquid leakage during the use can be prevented, so that accurate measurement is possible.

The pressing means that pressure is applied such that the film adheres to the substrate. The pressure may be applied from the film surface, may be applied from the substrate surface, or may be applied from both the film surface and the substrate surface (the surface to which the adhesive agent is not applied). The pressure is preferably applied from the substrate surface.

As long as the film is pressed onto the substrate, the pressure may be applied from the substrate surface (the surface to which the adhesive agent is not applied) in a state where the film surface is supported by a plate-like member or the like, or the pressure may be applied from the film surface in a state where the substrate surface (the surface to which the adhesive agent is not applied) is supported by a plate-like member or the like.

The applied pressure may be a pressure at a level at which the film can be pressed onto the substrate and at which the film and the substrate are not damaged. More specifically, the pressure is 32 to 643 kPa, preferably 129 to 321 kPa.

The subsequent step, that is, the adhesive agent curing step, may be carried out in the state where the film is pressed onto the substrate, that is, in the state where the pressure is applied. Alternatively, after pressing the film onto the substrate, the pressure may be removed (cancelled) before proceeding to the adhesive agent curing step.

Examples of the attachment step include a mode comprising the steps of: pressing the film onto the substrate at 32 to 643 kPa (preferably 129 to 321 kPa); and curing the adhesive agent in a state where the film is pressed onto the substrate at 32 to 643 kPa (preferably 129 to 321 kPa).

Another mode of the attachment step may be, for example, a mode comprising the steps of: pressing the film onto the substrate at 32 to 643 kPa (preferably 129 to 321 kPa) for a certain length of time; and thereafter, curing the adhesive agent in a state where the pressure applied to the film and the substrate is removed. In this case, the length of time for which the state under the application of the pressure is maintained is preferably not less than 3 seconds, more preferably not less than 10 seconds, especially preferably not less than 20 seconds, although the length of time may vary depending on the surface shape of the substrate and the type of the adhesive agent. As long as the adhesive agent does not flow into the flow path, there is no upper limit of the length of time of maintaining the pressure application. The upper limit may be, for example, 300 seconds or 100 seconds.

Preferably, in the application of the pressure to the film surface, the film surface is covered with an auxiliary sheet comprising an elastic material, and the pressure is applied through the auxiliary sheet. The pressure may be applied from the auxiliary sheet side, or may be applied from the substrate surface (the surface to which the adhesive agent is not applied).

Examples of the auxiliary sheet comprising an elastic material include rubber or silicone auxiliary sheets. Examples of the silicone include polydimethylsiloxane (PDMS). The auxiliary sheet may be constituted by an optically transparent material. The size of the auxiliary sheet is preferably a size that enables covering of the entire film surface. The thickness of the auxiliary sheet is not limited, and is preferably 1 to 3 mm from the viewpoint of operability.

More uniform attachment can be achieved by covering the film side with an auxiliary sheet comprising an elastic material, and applying pressure of a gas such as air from the auxiliary sheet side to let the film adhere to the substrate.

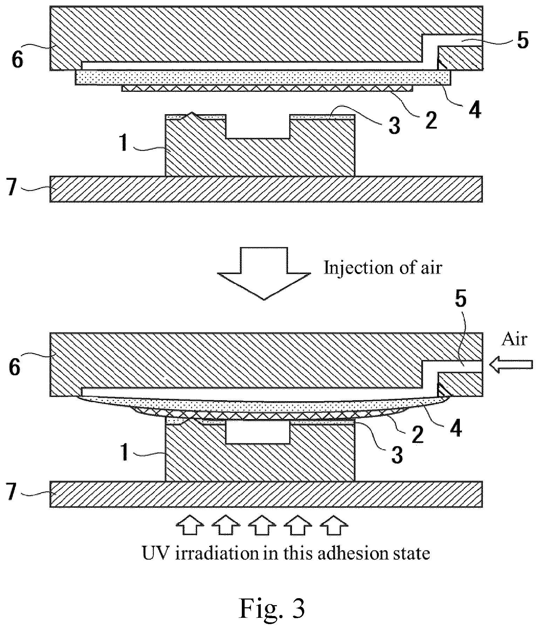

Fig. 3 illustrates one example of this embodiment.

In this embodiment, the step of attaching the film to the substrate is carried out using a member in which a cavity is formed, the member having a film-contacting surface (an auxiliary sheet 4) comprising an elastic material, which film-contacting surface inflates when a gas is injected from one end of the cavity 5. This member may be a member prepared by attaching a plate-like member 6 in which a cavity (air hole) through which a gas can pass is formed, to an auxiliary sheet 4 comprising an elastic material for covering the film surface, such that the gas can be injected into the member.

As illustrated in Fig. 3, the film-contacting surface of the member is brought into contact with the film surface of the microchip, and a gas is injected into the member to inflate the film-contacting surface. By this, pressure is applied to the film surface of the microchip. In this state, where the film is adhering to the substrate to which the adhesive agent is applied, the adhesive agent is cured to attach (bond) the film to the substrate. In Fig. 3, the microchip is supported by a supporting plate 7.

Here, in cases where the adhesive agent is a photocurable adhesive agent such as a UV-curable adhesive agent, and the member in which a cavity is formed, for example, the plate-like member and the elastic material, is constituted by an optically transparent material such as a UV-transparent member, the adhesive agent can be cured by irradiation with a light such as UV, to attach (bond) the film to the substrate. In cases where the substrate is an optically transparent material such as a UV-transparent member, the irradiation with the light such as UV may be carried out from the substrate side (the side not in contact with the film).

In cases where the adhesive agent is a thermosetting adhesive agent, the adhesive agent may be heated to cure in a state where pressure is applied by the gas, to achieve attachment (bonding) of the film to the substrate.

Another mode may be, for example, a mode in which the film is attached to the substrate using the adhesive agent in the step of attaching the film to the substrate, and, in this state, a sheet (an auxiliary sheet, which may also be referred to as elastic sheet) comprising an elastic material for covering the film surface is placed on the film side, followed by sandwiching the microchip from both the auxiliary sheet side and the substrate side using plate-like members, to thereby press the film onto the substrate.

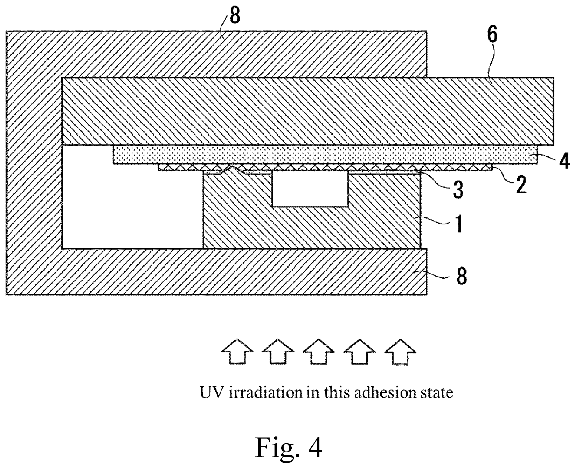

For example, as illustrated in Fig. 4, the film is pressed onto the substrate by sandwiching from the top and bottom of the substrate, that is, from both the side of the auxiliary sheet covering the film and the side of the substrate not in contact with the film, using plate-like members, and the adhesive agent is cured in this state by irradiation with a light such as UV By this, the film can be attached (bonded) to the substrate. Examples of the members include clip-type or clamp-type sandwiching members. The plate-like members and the auxiliary sheet are preferably made of an optically transparent material such as a UV-transparent member.

In cases where the adhesive agent is a thermosetting adhesive agent, the adhesive agent may be heated to cure in a state where pressure is applied by the plate-like members, to achieve the attachment (bonding) of the film to the substrate.

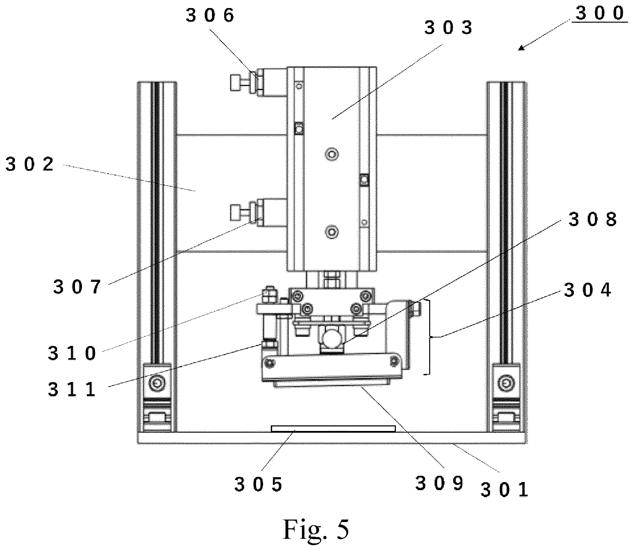

In another mode, a semi-automatic bonding jig as illustrated in Fig. 5 may be used in the step of attaching the film to the substrate, to enable stable preparation of homogeneous microchips.

The bonding jig 300 illustrated in Fig. 5 comprises a base plate 301, a gate-type support 302 fixed to the base plate 301, an air cylinder 303 fixed to the gate-type support 302, and a head 304 fixed to the air cylinder 303.

An elastic sheet 305 for fixing the film is placed on the base plate 301. The film is placed on the elastic sheet. In cases where the elastic sheet 305 is constituted by a resin or rubber having a high surface friction coefficient, such as polydimethylsiloxane (PDMS), displacement of the film can be prevented. Further, in cases where the elastic sheet 305 has a line or guide indicating the position where the film is to be preliminarily placed, the attachment of the film to the substrate can be carried out smoothly.

The gate-type support 302 comprises a plurality of vertical columns fixed to the base plate 301, and a beam fixed to the plurality of vertical columns.

The air cylinder 303 comprises an elevation speed controller 306 and a lowering speed controller 307. The air cylinder 303 is preferably a biaxial air cylinder with suppressed rotational deflection. The air cylinder 303 is fixed to the gate-type support 302, and used to move the head 304 upward and downward. The elevation speed controller 306 and the lowering speed controller 307 control the flow rate of a gas supplied to the air cylinder 303, to control the speed of upward and downward movement of the head 304.

The head 304 comprises an adsorption pad 308 for holding the substrate by adsorption, and a ceiling section 309. An angle adjustment screw 310 and an angle retention spring 311 may be provided in the head 304, to enable adjustment of the angle of the ceiling section 309. The adsorption pad 308 fixes the substrate to the head 304 by adsorption under reduced pressure. By adjusting the angle of the ceiling section 309, the angle between the film and the substrate to be attached to each other can be adjusted. In cases where the angle of the ceiling section 309 is adjusted, the angle is preferably adjusted to 0.5 to 3° with respect to the base plate 301. By this, the film can be more favorably attached to the substrate.

In cases where the head 304 is lowered in a state where the substrate is tilted with respect to the film, the substrate contacts the film at a certain angle as a result. Further lowering of the head 304 results in transmission of the stress to the angle retention spring 311 through the substrate and the ceiling section 309, causing compression of the angle retention spring 311. The substrate and the film finally become parallel to each other, and their complete attachment is achieved. Thus, by gradual application of pressure from one end to the other end of the substrate, air between the substrate and the film can be easily removed, so that better adhesion of the substrate to the film can be achieved. Further, by setting the lowering speed of the head 304 to 2 to 21 m/min, air between the substrate and the film can be more easily removed, so that excellent adhesion of the substrate to the film can be achieved.

In cases where a light irradiation device such as a UV irradiation device is placed under the bonding jig 300, and the base plate 301 is prepared with an optically transparent material, the adhesive agent can be cured by performing UV irradiation in a state where the substrate is pressed onto the film. By this, the process from the attachment to the bonding of the film to the substrate can be continuously carried out.

### <Curing Step>

In the curing step for bonding, curing means suitable for the type of the adhesive agent may be employed. For example, in cases where the adhesive agent is a photocurable adhesive agent such as a UV-curable adhesive agent, the film can be attached (bonded) to the substrate by curing the adhesive agent by irradiation with a light such as UV The wavelength of the irradiation light may be selected in accordance with the type of the adhesive agent. The irradiation time may also be appropriately selected. For example, the irradiation time may be selected such that the integrated amount of light is 500 to 5000 mW/cm².

In cases where the adhesive agent is a thermosetting adhesive agent, the adhesive agent can be cured by heating, to achieve the attachment (bonding) of the film to the substrate. The heating temperature may be selected in accordance with the type of the adhesive agent.

For improvement of the bonding strength between the film and the substrate, layering of the film on the substrate in the attachment step is preferably followed by (or carried out at the same time as) bonding of the outer periphery of the substrate and/or the periphery of the flow path using laser light irradiation or heat sealing. The outer periphery area of the substrate is, for example, an area of 0.5 to 1 mm from the outer periphery of the substrate toward the inside. The area is not limited, and may be adjusted in accordance of the substrate size and the flow path shape. In cases where such bonding is carried out, the adhesive agent is preferably applied to an area not subjected to the heat sealing, and is preferably not applied to the outer periphery of the substrate where welding or hot pressing is carried out. By this, a microchip having high bonding strength can be prepared without affecting the flow path shape. The bonding of the area close to the outer periphery by welding or hot pressing may be carried out at the same time as the pressing for bonding using the adhesive agent (for example UV irradiation), or may be carried out as a post-process.

In the bonding process, a plurality of substrates may be placed at precise positions on a film sheet having a large area, and welding or hot pressing may be carried out continuously or at the same time. In this case, microchips can be efficiently cut out from the film sheet by laser cutting or by use of a cutting blade after the bonding.

In cases where strong bonding of the periphery of the flow path by heat sealing or the like is carried out in combination with the bonding using a UV-curable adhesive agent, the heat-sealed portion can function as a support even when expansion or contraction occurs due to freezing or high temperature, so that the bond in the adhering area can be effectively maintained.

The present invention is described below concretely with reference to Examples, but the present invention is not limited to the following modes.

### EXAMPLE 1

A film was attached to a substrate having a surface to which a UV-curable adhesive agent was applied, to prepare the microchip shown in Fig. 1D.

### <Preparation of Microchips>

The substrate 201 shown in Fig. 1A (injection-molded article manufactured by MCC Advanced Moldings Co., Ltd.; COP resin) (size: 59.4 × 26.2 mm; thickness, 3 mm) was provided. In the substrate 201, the flow path 211 has a length of 33 mm, a depth of 80 µm, and a width of 1.2 mm in the inlet section or 0.3 mm in the narrow section; and the waste liquid storage section 212 has a length of 16 mm, a depth of 2.2 mm, and a width of 20 mm.

In the substrate 201, the hole corresponding to the inlet 213 was provided as a penetrating hole having a circular cross-sectional shape with an inner diameter of 2 mm. The hole corresponding to the air hole 214 was provided as a penetrating hole having a circular cross-sectional shape with an inner diameter of 0.5 mm. As the film 202, a COP film (size: 56.4 × 26.2 mm; thickness, 100 µm) was used.

The substrate 201 has projections with a height of about 10 to 20 µm due to a mold insert, at positions 3 mm distant toward the inside from both longer-side ends. The inner side of each projection is about 60 µm lower than the outer side of the projection. Along these steps of the substrate 201, air bubbles tend to enter the adhesive layer. An investigation was carried out in order to see whether adhesion of the film to the substrate in the vicinity of the steps is different between a case where the substrate is bonded to the film in a state where they are adhering to each other, and a case where the film is layered on the substrate using a roller (at a pressure of 400 kPa) followed by performing UV irradiation to allow bonding.

On the substrate 201, an adhesive agent was applied by screen printing to the area excluding the area corresponding to the flow path and the waste liquid storage section. The printing was carried out such that the film thickness of the adhesive agent was about 10 µm.

In the bonding, as shown in Fig. 3, a substrate 201 to which an adhesive agent 3 was applied was placed on an acrylic supporting plate 7 such that the adhesive agent surface faced upward, and a member prepared by bonding a plate-like member 6, in which a cavity was formed, to a silicone auxiliary sheet 4 was brought into contact with a film 2, which film 2 was placed such that its adhesion surface faced the adhesive surface of the substrate 201, to which the adhesive agent was applied. Thereafter, air was injected into the cavity 5 of the member to inflate the silicone auxiliary sheet 4, to apply pressure onto the film surface, to thereby make the film 2 adhere to the substrate 201. In this state, a light including an ultraviolet spectrum with a wavelength range of 200 to 450 nm was radiated from under the acrylic plate 7 to initiate curing reaction of the adhesive agent, to bond the film 2 to the substrate 201.

### <Evaluation of Microchip>

Distilled water was fed into the flow path of the prepared microchip. As a result, the distilled water was found to flow through only the flow path without showing leakage to the outside of the flow path. A decrease in the amount of air that had entered in the vicinity of the steps was found in the method where the bonding was carried out by performing UV irradiation in the state where the film was adhering to the substrate, compared to the case where the bonding was carried out by performing UV irradiation after layering the film on the substrate using a roller.

From these results, it was found that a microchip obtained by attaching the substrate to the film and curing the adhesive agent in a state where pressure is applied to the film surface can achieve adhesion of the film to the substrate even when a projection is present on the substrate surface. Thus, even in cases where a chip having a projection in the periphery of the flow path is to be bonded, entrance of air in the vicinity of the projection can be suppressed by the curing of the adhesive agent in the state where pressure is applied to the substrate and the film, so that a chip which is less likely to suffer from liquid leakage can be produced.

### EXAMPLE 2

### <Preparation of Microchip>

The direction of the application of the pressure in the step of bonding the substrate to the film was studied. The same substrate, film, adhesive agent, and application conditions as in Example 1 were used. When the pressure was applied from the film side, the bonding was possible by Example 1.

In order to investigate whether the bonding is possible by application of pressure from the substrate side, a trial preparation was carried out using the bonding jig illustrated in Fig. 5. In order to investigate whether the bonding is possible by application of pressure from both the substrate side and the film side, trial preparation was carried out using a clamp capable of application of pressure from the both sides as illustrated in Fig. 4. The materials used and the process until the application of the adhesive agent to the substrate were the same as in Example 1.

In the case where the bonding jig illustrated in Fig. 5 was used, the angle of the ceiling section 309 was 0.5° when the jig was not adhering to the film, and the lowering speed of the ceiling section 309 was 4 m/min. The film was held on the elastic sheet placed on the base plate 301, and the substrate to which the adhesive agent was applied was held on the ceiling section 309 by adsorption such that the bonding surface faced the base plate 301 side. Thereafter, the ceiling section 309 was lowered to make the substrate adhere to the film, and a light including an ultraviolet spectrum with a wavelength range of 200 to 450 nm was radiated to initiate curing reaction of the adhesive agent, to bond the film 2 to the substrate 201.

In the case where the clamp illustrated in Fig. 4 was used, transparent acrylic plates were placed on both jaw parts of the clamp, which were to be in contact with the materials, and further, elastic sheets were attached to the acrylic parts. The substrate to which the adhesive agent was applied was held on one side, and the film was held on the other side by adsorption. Thereafter, the clamp was tightened to make the substrate adhere to the film, and the film 2 was bonded to the substrate 201 by UV irradiation.

### <Evaluation of Microchip>

Distilled water was fed into the flow path of each prepared microchip. As a result, in any of the microchips prepared by the bonding under the above conditions, the distilled water was found to flow through only the flow path groove without showing leakage to the outside of the flow path. Since the UV irradiation was performed in the state where the film was adhering to the substrate, the amount of air that had entered was found to be decreased. Thus, it was found that favorable bonding can be achieved by application of pressure from any of the film side, substrate side, or both sides.

### EXAMPLE 3

### <Preparation of Microchip>

Appropriate pressure conditions for adhesion between the substrate and the film were studied. The same substrate, film, adhesive agent, and application conditions as in Example 1 were used. Bonding was carried out using a vise capable of application of a load to a sample by lowering of a ceiling section in the longitudinal direction from the top side toward the bottom side. The substrate and the film were placed in the vise such that their bonding surfaces faced each other. More specifically, the substrate was held in the stage side of the vise, and a silicone sheet that is an elastic sheet having a thickness of 1 mm was placed on the ceiling section, wherein the film was immobilized on the silicone sheet. By lowering the ceiling section in this state, pressurization was performed from the film side, to carry out the bonding. A digital weight scale was placed immediately under the stage to enable reading of a load (kg) that reflects the level of tightening of the vise. The unit of the load was converted to newton (N) by multiplying the read load by 10, and then the obtained value (N) was divided by the area of the substrate to calculate the pressure (Pa). In the following description, kPa is used as the unit. After the adhesion of the substrate to the film, the pressure was removed to 0 kPa. Thereafter, the amount of air that had entered the adhesive agent layer, and the like were observed.

For studying the conditions, bonding was carried out at a load of each of 5 kg, 10 kg, 20 kg, 30 kg, 50 kg, 100 kg, 150 kg, and 200 kg. These loads can be converted to pressures of about 32 kPa, 64 kPa, 129 kPa, 193 kPa, 321 kPa, 643 kPa, 964 kPa, and 1285 kPa, respectively. The length of time for which the application of the load was maintained was 3 seconds.

### <Evaluation of Microchip>

As a result of the experiment, in the case of pressurization at 32 kPa, the film adhered to the outer side of the projection of the substrate 201. However, uneven adhesion of the film was found in the inner side including the vicinity of the flow path, where the height was about 60 µm lower than the outer side. In the case of pressurization at 64 kPa, adhesion was achieved in the vicinity of the flow path, but entrance of air bubbles into the adhesive agent layer was found along the steps, leading to a concern that the peel strength may decrease due to a decrease in the adhering area. In the cases of pressurization at 129 kPa, 193 kPa, 321 kPa, or 643 kPa, entrance of air bubbles into the adhesive agent layer was not found, and favorable bonding was possible. In the cases of pressurization at 964 kPa or 1285 kPa, severe bonding defects occurred in the vicinity of the outer periphery of the substrate 201 and the vicinity of the waste liquid storage section. Since these defects cause leakage of a liquid sample from the waste liquid storage section to the outside, such pressurization was not suitable for the preparation of a microchip. From these results, it was found that the adhesion of the substrate to the film through the adhesive agent can be achieved preferably at a pressure of 32 to 643 kPa, more preferably at a pressure of 129 to 643 kPa.

### EXAMPLE 4

### <Preparation of Microchip>

An investigation was carried out to see how the quality of bonding is affected by the presence or absence of an elastic sheet(s) during the adhesion of the substrate to the film. The same substrate, film, adhesive agent, and application conditions as in Example 1 were used. As the elastic sheet, a silicone sheet having a thickness of 1 mm was used. For bonding of the substrate to the film, the same vise as in Example 3 was used. Bonding tests were carried out using the vise under a condition where elastic sheets were placed on both the stage side holding the substrate and the ceiling section holding the film, under a condition where no elastic sheet was placed on either side, under a condition where an elastic sheet was placed only on the stage side, and under a condition where an elastic sheet was placed only on the ceiling section. Observation of the obtained microchips were then carried out. The pressure applied for the adhesion of the substrate to the film was 129 kPa. Each microchip was kept under the pressure for 30 seconds, and then the bonded microchip was observed. For the condition where elastic sheets were placed on both the stage side holding the substrate and the ceiling section holding the film, and the condition where an elastic sheet was placed only on the ceiling section, pressure distribution on the substrate was observed using a prescale for ultralow pressure (Fujifilm Corporation), which is a pressure-sensitive paper. The prescale was cut into the same size as the substrate, and placed such that it was sandwiched between the substrate and the film for which adhesion was to be investigated. After pressurization, the prescale shows a red color having a color density that depends on the pressure applied. The pressure distribution was thus observed using the color as an index.

### <Evaluation of Microchip>

As a result of the experiment, the condition where elastic sheets were placed on both the stage side holding the substrate and the ceiling section holding the film led to formation of a loose part in the vicinity of the outer periphery of the substrate, so that there was a concern that the substrate may be detached from the film when a force is applied. The condition where no elastic sheet was placed on either the stage side holding the substrate or the ceiling section holding the film, and the condition where an elastic sheet was placed only on the stage side holding the substrate, failed to achieve adhesion of the entire area in the inner side of the substrate including the vicinity of the flow path, so that introduction of a liquid sample was impossible. When observation of the pressure distribution using the pressure-sensitive paper prescale was carried out for the condition where no elastic sheet was placed on either side, only the portions outside the steps formed by the insert were found to have undergone color change into red, showing a frame-like pattern, while the inner-side portion, which is lower than the outer side, did not show color change, indicating that pressure had been hardly applied thereto. In contrast, the condition where an elastic sheet was placed only on the ceiling section holding the film led to occurrence of no loose part in any area on the substrate, so that favorable bonding was possible. In the measurement of the pressure distribution using the prescale, color change into red was found throughout the area corresponding to the entire area of the substrate, indicating the pressure had been uniformly applied. From these results, it was found that, by placing an elastic sheet in the side holding the film, adhesion between the film and the substrate can be improved to enable favorable bonding. This is thought to be because a thin film can be made capable of following irregularity of the substrate surface when the elastic sheet is present therebetween. It is thought, on the other hand, that placement of elastic sheets on both the film side and the substrate side may cause loss of the applied pressure, which is likely to result in uneven adhesion.

### EXAMPLE 5

### <Preparation of Microchip>

The length of time for which the pressure application is to be maintained for achieving adhesion between the substrate and the film was studied. Based on the result of Example 3, it was found that, at a pressure suitable for allowing adhesion between the substrate and the film through the adhesive agent, that is, at a pressure of 129 kPa to 643 kPa, the adhesion can be sufficiently achieved by maintaining the pressure application for about 3 seconds. However, as can be seen from the fact that severe bonding defects occurred in the vicinity of the waste liquid storage section when a pressure of 964 kPa or 1285 kPa was applied to perform the adhesion in Example 3, application of an increased pressure may cause bonding defects when a microchip is produced by bonding a thin material such as a film to a substrate having a large area. In view of this, investigation was carried out to see whether or not favorite bonding can be achieved even by application of a pressure of 32 kPa or 64 kPa in cases where the length of time for which the pressure application is maintained is adjusted. The same substrate, film, adhesive agent, and application conditions as in Example 1 were used. For bonding of the substrate to the film, the same vise as in Example 3 was used. As the elastic sheet, a silicone sheet having a thickness of 1 mm was used. The elastic sheet was placed only on the ceiling section side holding the film. The length of time for which the pressure application is maintained was set to 3 seconds, 10 seconds, 30 seconds, and 1 minute in the test.

### <Evaluation of Microchip>

As a result of the experiment, in the cases where a pressure of 32 kPa or 64 kPa was applied, and the length of time for which the pressure application was maintained was 3 seconds, adhesion could be achieved in the vicinity of the flow path, but entrance of air bubbles into the adhesive agent layer was found along the steps, leading to a concern that the peel strength may decrease due to a decrease in the adhering area. However, when the length of time for which the pressure application was maintained was 10 seconds, 30 seconds, or 1 minute, favorable adhesion could be achieved in the areas including the steps and the vicinity of the waste liquid storage section, and qualities equivalent to that of the microchip prepared by pressurization at 129 kPa could be obtained. From these results, it was found that favorable adhesion between the substrate and the film can be achieved even at a pressure of 32 kPa or 64 kPa when the length of time for which the pressure application is maintained is not less than 10 seconds. The upper limit of the length of time for which the pressure application is maintained depends on the shape of the flow path and the type of the adhesive agent. However, the length of time is not limited as long as the adhesive agent does not flow into the flow path.

### EXAMPLE 6

### <Preparation of Microchip>

Appropriate conditions for the use of the bonding jig illustrated in Fig. 5 were studied in terms of the angle of the ceiling section 309 and the lowering speed of the ceiling section 309. The same substrate, film, adhesive agent, and application conditions as in Example 1 were used. The pressure for the bonding by the bonding jig was set to 400 kPa, and the lowering speed of the ceiling section 309 was set to 4 m/min. The substrate to which the adhesive agent was applied was placed on the ceiling section 309 of the bonding jig such that the surface to which the adhesive agent was applied faced downward, and the film was placed on the base plate 301 such that the bonding surface faced upward. For determination of an appropriate angle of the ceiling section 309, the substrate was bonded to the film after setting the angle of the ceiling section 309 to the base plate 301 to 0°, 0.25°, 0.5°, 1°, 1.5°, 2°, 3°, 4°, or 5°. Favorable angle conditions were determined based on the area ratio of loose parts formed in each chip. In the calculation of the area ratio of loose parts, a photograph of the bonded chip was taken, and the image was captured and digitized, followed by calculating the pixel numbers of the sites with occurrence of a loose part and the sites with occurrence of no loose part. Thereafter, the pixel number of the sites with occurrence of a loose part was divided by the pixel number of the sites with occurrence of no loose part, to calculate the area ratio of loose parts.

### <Evaluation of Microchip>

As a result of the experiment, the chip prepared by bonding at an angle of 0° showed an area ratio of loose parts of 5.8%. Since the chip prepared by bonding at an angle of 0.25° showed an area ratio of loose parts of 3.2%, it was found that the area ratio of loose parts can be reduced by setting an angle for the ceiling section. In the chips prepared by bonding at 0.5°, 1°, 1.5°, 2°, or 3°, the area ratios of loose parts were 2%, 2.5%, 3.4%, 3%, and 3%, respectively, indicating favorable bonding results. In the chips prepared by bonding at 4° or 5°, displacement occurred between the substrate and the film at the time when the substrate and the film contacted each other to become horizontal, causing the adhesive agent to flow into the flow path. Both cases resulted in an area ratio of loose parts of 4.3%, which was higher than those in the results obtained at the angles of 0.25 to 3°.

These results indicate that, in the case of utilization of a mechanism in which an angle is set for the side where the substrate is fixed, and in which the substrate and the film gradually become horizontal by the contact therebetween, the angle of the substrate side is preferably 0.25 to 3°.

### EXAMPLE 7

### <Preparation of Microchip>

Appropriate conditions for the use of the bonding jig illustrated in Fig. 5 were studied in terms of the lowering speed of the ceiling section 309. The same substrate, film, adhesive agent, and application conditions as in Example 1 were used. The pressure for the bonding by the bonding jig was set to 400 kPa, and the angle of the ceiling section 309 to the base plate 301 was set to 0.5°. By adjusting the lowering speed using the lowering speed controller, the substrate was bonded to the film at 2, 4, 8, 15, 21, 23, 24, or 40 m/min, and favorable lowering speed conditions were determined based on the area ratio of loose parts formed in the chip.

### <Evaluation of Microchip>

In the experiment, all of the chips prepared by bonding at a lowering speed of 2, 4, 8, 15, or 21 m/min showed favorable results with area ratios of loose parts of 2 to 3%. However, the chips prepared by bonding at a lowering speed of 23 or 40 m/min showed slight formation of loose parts around the center of the chip. This is thought to be because the time for allowing the air to escape during the bonding could not be secured due to the increased lowering speed. These results indicate that, in the case of utilization of a mechanism in which an angle is set for the side where the substrate is fixed, and in which the substrate and the film gradually become horizontal by the contact therebetween, the lowering speed of the substrate side is preferably 2 to 21 m/min.

### DESCRIPTION OF SYMBOLS

1 ... Substrate; 2 ... Film; 3 ... Adhesive agent; 4 ... Auxiliary sheet; 5 ... Cavity; 6 ... Plate-like member; 7 ... Supporting plate; 8 ... Sandwiching member; 200 ... Microchip; 201 ... Substrate; 211 ... Flow path; 212 ... Waste liquid storage section; 213 ... Inlet; 214 ... Air hole; 202 ... Film; 300 ... Bonding jig; 301 ... Base plate; 302 ... Gate-type support; 303 ... Air cylinder; 304 ... Head; 305 ... Elastic sheet; 306 ... Elevation speed controller; 307 ... Processing-side speed controller; 308 ... Adsorption pad; 309 ... Ceiling section; 310 ... Angle adjustment screw; 311 ... Angle retention spring

## Claims

1. A method for manufacturing a microchip for liquid sample analysis, the microchip comprising a flow path therein, the method comprising:
a step of providing a substrate comprising on a surface thereof a groove serving as the flow path;
a step of providing a film capable of forming the flow path by covering the surface of the substrate to seal the groove;
a step of applying an adhesive agent and/or a gluing agent to the surface of the substrate on which the groove is formed; and
a step of attaching the film on the substrate in such a manner that the film and the surface of the substrate on which the adhesive agent and/or the gluing agent is/are applied overlap each other; and
wherein the step of attaching comprises
a step of pressing the film onto the substrate at 32 to 643 kPa, and
a step of curing the adhesive agent and/or the gluing agent.

2. The method according to claim 1, wherein the step of attaching comprises
a step of pressing the film onto the substrate at 32 to 643 kPa; and
a step of curing the adhesive agent and/or the gluing agent in a state where the film is pressed onto the substrate at 32 to 643 kPa.

3. The method according to claim 1, wherein the step of attaching comprises
a step of pressing the film onto the substrate at 32 to 643 kPa for a certain length of time, and thereafter, curing the adhesive agent and/or the gluing agent in a state where the pressure applied to the film and the substrate is removed.

4. The method according to claim 3, wherein the step of attaching comprises
a step of pressing the film onto the substrate at 32 to 128 kPa for not less than 10 seconds, and thereafter, curing the adhesive agent and/or the gluing agent in a state where the pressure applied to the film and the substrate is removed.

5. The method according to claim 3, wherein the step of attaching comprises
a step of pressing the film onto the substrate at 129 to 643 kPa for not less than 3 seconds, and thereafter, curing the adhesive agent and/or the gluing agent in a state where the pressure applied to the film and the substrate is removed.

6. The method according to any one of claims 1 to 5, wherein the step of pressing the film onto the substrate uses a member in which a cavity is formed, the member having a film-contacting surface comprising an elastic material, which film-contacting surface inflates when a gas is injected from one end of the cavity, wherein the step is carried out by bringing the film-contacting surface of the member into contact with the film surface of the microchip and injecting the gas into the member to apply pressure onto the film surface of the microchip.

7. The method according to claim 6, wherein the member in which a cavity is formed is a member prepared by attaching a plate-like member to a sheet comprising an elastic material for covering the film surface, in such a manner that a gas can be injected into the member.

8. The method according to any one of claims 1 to 7, wherein the step of pressing the film onto the substrate is carried out by applying pressure to an auxiliary sheet comprising an elastic material for covering the film surface, the auxiliary sheet being placed in the film side.

9. The method according to claim 6 or 7, wherein the elastic material is rubber or silicone.

10. The method according to any one of claims 6 to 8, wherein the adhesive agent and/or the gluing agent is/are a photocurable adhesive agent and/or a photocurable gluing agent, and the member in which a cavity is formed or the auxiliary sheet used in the step of attaching is constituted by an optically transparent material.

11. The method according to claim 10, wherein the photocurable adhesive agent and/or the photocurable gluing agent is/are a UV-curable adhesive agent and/or a UV-curable gluing agent, and the optically transparent material is a UV-transparent material.
